# EUROPÄISCHE PATENTSCHRIFT

(11) **EP 2 681 434 B1**
(45) Veröffentlichungstag und Bekanntmachung des Hinweises auf die Patenterteilung: **01.02.2017**
(21) Anmeldenummer: 12703067.4
(22) Anmeldetag: 02.02.2012
(51) Int. Cl.: F02D 19/06

(54) **VERFAHREN ZUM BESTIMMEN EINER EIGENSCHAFT EINES KRAFTSTOFFS**
METHOD FOR DETERMINING A PROPERTY OF A FUEL
PROCÉDÉ POUR DÉTERMINER UNE CARACTÉRISTIQUE D'UN CARBURANT

(30) Priorität: 04.03.2011 DE 102011005141
(43) Veröffentlichungstag der Anmeldung: 08.01.2014
(73) Patentinhaber: Robert Bosch GmbH, 70442 Stuttgart (DE)
(72) Erfinder: SIEDENTOPF, Matthias, 71296 Heimsheim (DE); SPINDLER, Susanne, 70197 Stuttgart (DE); HORN, Matthias, 71691 Freiberg (DE); RETTICH, Andreas, 71083 Herrenberg-Kuppingen (DE); HOFFMANN, Florian, 71638 Ludwigsburg (DE)
(86) Internationale Anmeldenummer: PCT/EP2012/051749
(87) Internationale Veröffentlichungsnummer: WO 2012/119819

(56) Entgegenhaltungen:
- DE-A1- 10 252 476
- GB-A- 2 345 090
- US-A- 4 594 979
- US-A- 4 955 345

## Beschreibung

Die Erfindung betrifft ein Verfahren zum Bestimmen mindestens einer Eigenschaft eines Kraftstoffs, ein Verfahren zum Betreiben eines Verbrennungsmotors und eine Anordnung zur Durchführung der Verfahren.

### Stand der Technik

Die Einspritzmenge bei Common Rail Systemen (CRS) ist unter anderem von verschiedenen Kraftstoffeigenschaften abhängig, die wiederum von der Art des Kraftstoffs und den Umgebungsbedingungen abhängen. Beispielsweise ist die Viskosität der Kraftstoffsorte, bspw. Winterdiesel, Arcticdiesel, Biodiesel, Mischungen verschiedener Kraftstoffsorten, und zusätzlich auch von der Temperatur abhängig. Solange im CRS keine Möglichkeit besteht, wichtige Kraftstoffeigenschaften, z. B. über einen Sensor, zu bestimmen, können Mengeneinflüsse dieser Eigenschaften nur ungenügend oder überhaupt nicht korrigiert werden.

Es ist zu beachten, dass für einen unterhalb des Gefrierpunkts durchzuführenden Kaltstart üblicherweise größere Startzeiten als bei einem Start oberhalb des Gefrierpunkts zulässig sind. Hierbei variieren die Anforderungen bezüglich der Startzeit bei einer minimalen Temperatur. Es wird angestrebt, eine Startzeit, d. h. ein Zeitintervall bis zum Ende des Startzustands, auch bei einem Kaltstart zu verkürzen.

Die DE 102 52 476 A1 zeigt ein Verfahren zur Bestimmung der Kraftstoffart. Während des Betriebes der Brennkraftmaschine wird ein Magnetventil betätigt und der Zeitpunkt des lokalen Minimums des Stroms bestimmt. Diese lokale Minimum kennzeichnet, den Öffnungszeitpunkt des Magnetventils. Ausgehend von dem Vergleich mit einem gespeicherten Zeitpunkt wird die Kraftstoffart bestimmt.

Eine Vorrichtung zur Bestimmung von Eigenschaften eines Brennstoffs einer Dieselmaschine ist aus der Druckschrift DE 196 44 102 A1 bekannt. Hierbei ist vorgesehen, dass als Eigenschaft des Brennstoffs insbesondere dessen Viskosität bestimmt wird. Hierbei werden ein Startzeitpunkt für eine Brennstoffzufuhr eines Pumpenkolbens an eine Einspritzdüse und ein Startzeitpunkt der Brennstoffeinspritzung der Einspritzdüse abgetastet. Eine Differenz zwischen dem Startzeitpunkt für die Brennstoffzufuhr und dem Startzeitpunkt für die Brennstoffeinspritzung wird berechnet und als Einspritzverzögerungsperiode betrachtet.

Eine Einspritztaktsteuerung für Dieselmotorbrennstoff ist aus der Druckschrift DE 43 23 967 A1 bekannt. Dabei wird ein Steuerdruck eines einzuspritzenden Brennstoffs durch ein Steuerventil entsprechend seiner Öffnung eingestellt. Eine Viskosität des verwendeten Brennstoffs wird aus einem Einschaltverhältnis eines Impulssignals, das dem Steuerventil zugeführt wird, berechnet.

Ein Verfahren zum Betreiben eines hydraulisch beaufschlagten Kraftstoffeinspritzsystems ist in der Druckschrift US 5 357 912 beschrieben. Hierbei werden ein Kennfeld und/oder Gleichungen zur Kontrolle einer Einspritzdüse verwendet. Aus dem Kennfeld und/oder den Gleichungen werden die Transporteigenschaften in Abhängigkeit eines Drucks sowie einer Viskosität des Kraftstoffs bestimmt.

### Offenbarung der Erfindung

Vor diesem Hintergrund werden ein Verfahren zum Bestimmen mindestens einer Eigenschaft eines Kraftstoffs, ein Verfahren zum Betreiben eines Verbrennungsmotors und eine Anordnung mit den Merkmalen der unabhängigen Patentansprüche vorgestellt. Weitere Ausgestaltungen der Erfindung ergeben sich aus den abhängigen Patentansprüchen und der Beschreibung.

Es wird somit ein Verfahren vorgestellt, das mit Hilfe vorhandener Komponenten, d.h. ohne dass ein zusätzlicher Sensor erforderlich ist, Kraftstoffeigenschaften, wie insbesondere die Viskosität, bestimmt. Diese Kraftstoffeigenschaften beeinflussen das dynamische Verhalten des durch den Kraftstoff bewegten Ankers und damit auch die Schließdauer.

Das Verfahren wird bei einem Magnetventil, insbesondere bei einem Magnetventil eines Common-Rail-Aktors, eingesetzt, wobei mit dem Verfahren z.B. eine Optimierung bei Kaltstart erreicht werden kann. Weiterhin können Komponenten der gesamten Anlage geschützt werden.

Das Magnetventil, das sich im Kraftstoff befindet, weist einen Magnetanker auf, der sich durch den Kraftstoff bewegt. Die Viskosität des Kraftstoffs wirkt sich dabei auf das dynamische Verhalten des Magnetankers aus. Damit besteht ein Zusammenhang zwischen Schließdauer des Magnetankers und der Viskosität des Kraftstoffs.

Die Schließdauer kann bei einer Ansteuerdauer oder bei mehreren Ansteuerdauern gemessen werden. Werden bspw. zwei Messungen durchgeführt, kann die ermittelte Differenz zwischen den beiden Ansteuerdauern mittels mathematischer Methoden weiterverarbeitet und ausgewertet werden.

Auf diese Weise werden Kraftstoffeigenschaften bestimmt, wie bspw. die Viskosität. Daraus können spezifische Korrekturwerte für die Ansteuerdauer ermittelt werden. Ziel ist dabei die Gleichstellung der Einspritzmenge unabhängig von den Kraftstoffeigenschaften, wie z.B. der Viskosität.

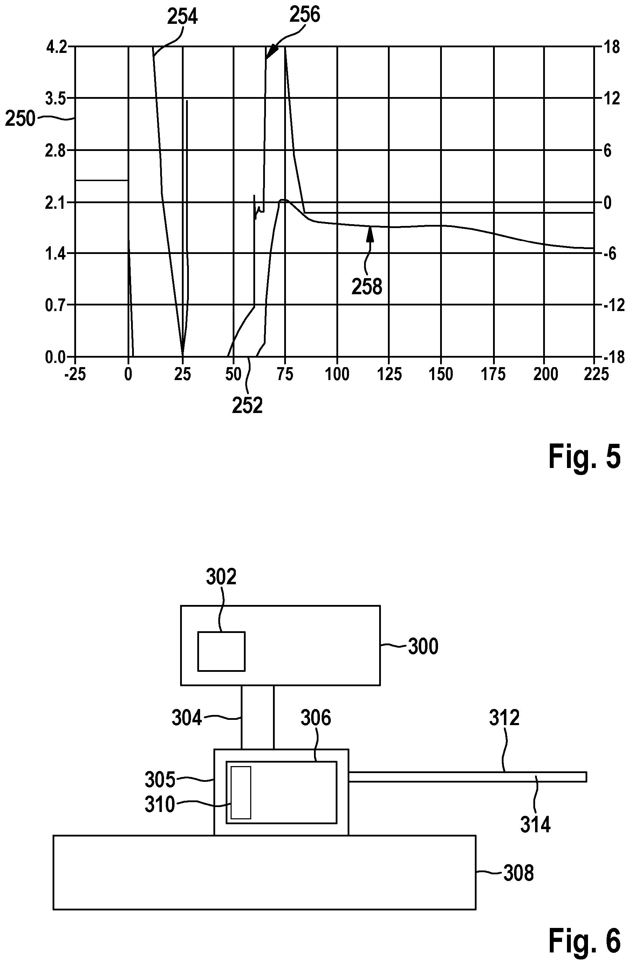

Die Messungen basieren auf der Funktion VCA/VCC (valve close adaption/valve close control). Dabei wird mittels eines Sensorstroms die Schließdauer des Magnetankers bestimmt. Dieses Verfahren wird unter Bezugnahme auf Figur 5 beschrieben.

Die Schließdauer des Magnetankers ist definiert als die Zeit von Beginn der Stromlöschung des Magnets bis zum Einschlag des Magnetankers im Ventilsitz. Der Zeitpunkt des Einschlags wird dadurch bestimmt, dass, nachdem der Strom in der Spule den Wert Null erreicht und nach einem definierten Zeitraum, der bspw. zwischen 10 und 200 µs liegen kann, die Magnetventilspule mit einem Sensorstrom bestromt wird. Der Sensorstrom erfährt aufgrund der Induktivitätsänderung, ausgelöst durch die Bewegung des Ankers im Magnetfeld, eine Änderung. Diese Änderung des Sensorstroms kann bei einer signifikanten Änderung der Geschwindigkeit, z.B. durch den Einschlag des Ankers im Sitz, eindeutig gemessen werden. Somit kann die Zeit bestimmt werden, die der Anker zum Schließen benötigt, nämlich die Schließdauer. Auf Grundlage der gemessenen Schließdauer kann dann ein Faktor bzw. eine Größe ermittelt werden, der bzw. die die mindestens eine Eigenschaft, insbesondere die Viskosität, des Kraftstoffs repräsentiert.

Kraftstoffeigenschaften umfassen neben der Viskosität eine Zusammensetzung des Kraftstoffs, d. h. einen Gehalt an verschiedenen Kraftstoffkomponenten und somit eine Art des Kraftstoffs, und Fließeigenschaften, die sich aus der Zusammensetzung des Kraftstoffs sowie abhängig von Umgebungseinflüssen, wie bspw. der Temperatur, ergeben. Wenn die Kraftstoffeigenschaften bekannt sind, ist es möglich, einen Betrieb des Verbrennungsmotors an die Kraftstoffeigenschaften und somit die Zusammensetzung des Kraftstoffs unter Berücksichtigung der Umgebungseinflüsse anzupassen.

Durch die Erfindung kann u. a. die Mengenungenauigkeit bei Variation von Kraftstoffeigenschaften verringert werden, ohne zusätzliche Sensorik in der Einspritzanlage integrieren zu müssen.

Das Verfahren zum Betreiben eines Verbrennungsmotors nutzt das vorstehend beschriebene Verfahren, wobei mindestens ein Betriebsparameter einer Einspritzanlage, die ein Magnetventil umfasst, des Verbrennungsmotors in Abhängigkeit der ermittelten Eigenschaft, insbesondere der Viskosität des Kraftstoffs, eingestellt wird.

Die erfindungsgemäße Anordnung ist dazu ausgebildet, sämtliche Schritte des vorgestellten Verfahrens zum Bestimmen mindestens einer Eigenschaft eines Kraftstoffs durchzuführen. Dabei können einzelne Schritte dieses Verfahrens auch von einzelnen Komponenten der Anordnung durchgeführt werden. Weiterhin können Funktionen der Anordnung oder Funktionen von einzelnen Komponenten der Anordnung als Schritte des Verfahrens zum Bestimmen mindestens einer Eigenschaft umgesetzt werden. Außerdem ist es möglich, dass Schritte des Verfahrens als Funktionen wenigstens einer Komponente der Anordnung oder der gesamten Anordnung realisiert werden. Die durch das Verfahren zum Bestimmen der mindestens einen Eigenschaft und durch die Anordnung bestimmte Eigenschaft des Kraftstoffs kann weiterhin für das Verfahren zum Betreiben des Verbrennungsmotors verwendet werden.

Weitere Vorteile und Ausgestaltungen der Erfindung ergeben sich aus der Beschreibung und den beiliegenden Zeichnungen.

Es versteht sich, dass die voranstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Kurze Beschreibung der Zeichnungen
- Figur 1: zeigt in einem Graphen Beispiele für erste Kennlinien von Parametern für Einspritzungen, die sich für zwei verschiedene Kraftstoffe ergeben.
- Figur 2: zeigt in einem Graphen die Temperaturabhängigkeit der Viskosität verschiedener Kraftstoffe.
- Figur 3: zeigt in einem Graphen Zusammenhänge zwischen einer Schließdauerdifferenz und einer kinematischen Viskosität, die sich aus Messwerten für unterschiedliche Kraftstoffsorten bei verschiedenen Temperaturen ergeben.
- Figur 4: zeigt in einem Graphen Zusammenhänge zwischen Einspritzmenge und kinematischer Viskosität.
- Figur 5: zeigt in einem Graphen den Verlauf des Sensorstroms.
- Figur 6: zeigt eine Ausführung der Anordnung zur Durchführung des beschriebenen Verfahrens.

### Ausführungsformen der Erfindung

Die Erfindung ist anhand von Ausführungsformen in den Zeichnungen schematisch dargestellt und wird nachfolgend unter Bezugnahme auf die Zeichnungen beschrieben. Zu beachten ist, dass in den Zeichnungen und der Beschreibung genannte Werte und Wertebereiche lediglich beispielhaft angegeben sind.

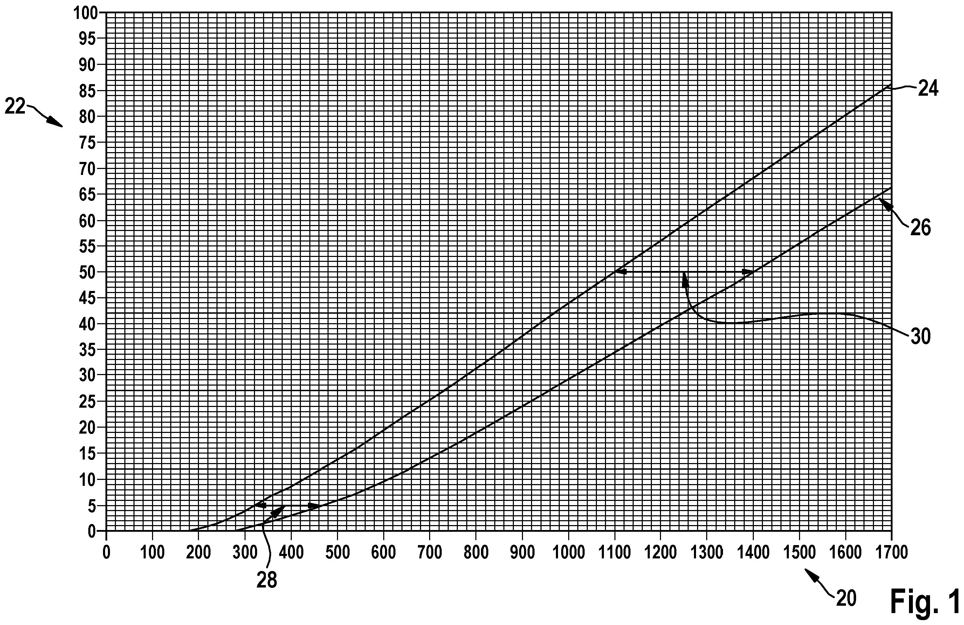

In dem Diagramm aus Figur 1 ist entlang einer Abszisse 20 eine Ansteuerdauer in µs und entlang einer Ordinate 22 eine Einspritzmenge für einen Kraftstoff in mm³ aufgetragen. In dem Diagramm aus Figur 1 ist eine erste Kurve 24 für die Einspritzmenge in Abhängigkeit der Ansteuerdauer für sogenannten Arcticdiesel, der üblicherweise für tiefe Temperaturen geeignet ist, und eine zweite Kurve 26 für einen sogenannten Winterdiesel dargestellt.

Durch zwei Doppelpfeile 28, 30 sind Unterschiede von Ansteuerdauern als dynamische Eigenschaft eines Einspritzventils, bspw. eines Magnetventils, bei konstanten Einspritzmengen für die beiden Kraftstoffsorten dargestellt. So variiert bei einer Einspritzmenge von 5 mm³ eine Ansteuerdauer um ca. 140 µs (erster Doppelpfeil 28). Die Unterschiede zwischen den Ansteuerdauern nehmen mit zunehmender Einspritzmenge zu. So unterscheiden sich die Ansteuerdauern der beiden Kraftstoffsorten bei einer Einspritzmenge von 50 mm³ um ca. 310 µs (zweiter Doppelpfeil 130).

Die gezeigten Kurven 24, 26 wurden aus Mittelwerten von mehreren Kältezellenversuchen bei einem Raildruck von 600 bar und somit einem Druck der genannten Kraftstoffsorten in einem Kraftstoffspeicher einer Einspritzanlage in diesem Fall bei jeweils -25 °C ermittelt.

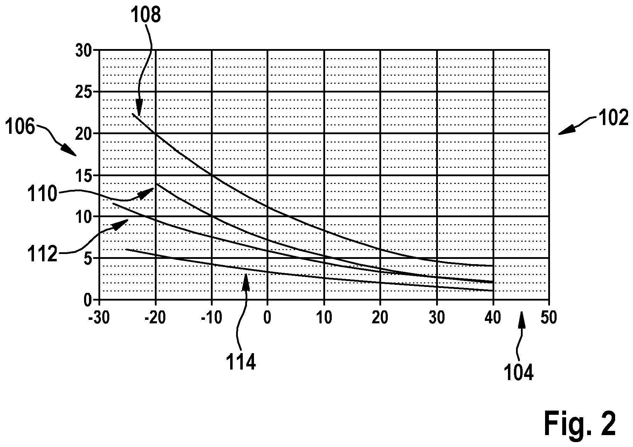

Figur 2 zeigt ein Diagramm 102 mit einer Abszisse 104, entlang der eine Temperatur in °C aufgetragen ist, und einer Ordinate 106, entlang der eine kinematische Viskosität eines Kraftstoffs in mm²/s aufgetragen ist. Das Diagramm umfasst vier Kurven 108, 110, 112, 114. Dabei stellt eine erste Kurve 108 eine temperaturabhängige kinematische Viskosität eines ersten Winterdieselkraftstoffs, eine zweite Kurve 110 eine temperaturabhängige kinematische Viskosität eines Testdiesels, eine dritte Kurve 112 eine temperaturabhängige kinematische Viskosität eines Winter-Dieselkraftstoffs und eine vierte Kurve 114 eine temperaturabhängige kinematische Viskosität eines sogenannten Arctic-Dieselkraftstoffs dar. Somit zeigt das Diagramm 102 aus Figur eine Temperaturabhängigkeit von kinematischen Viskositäten für verschiedene Winterdieselsorten.

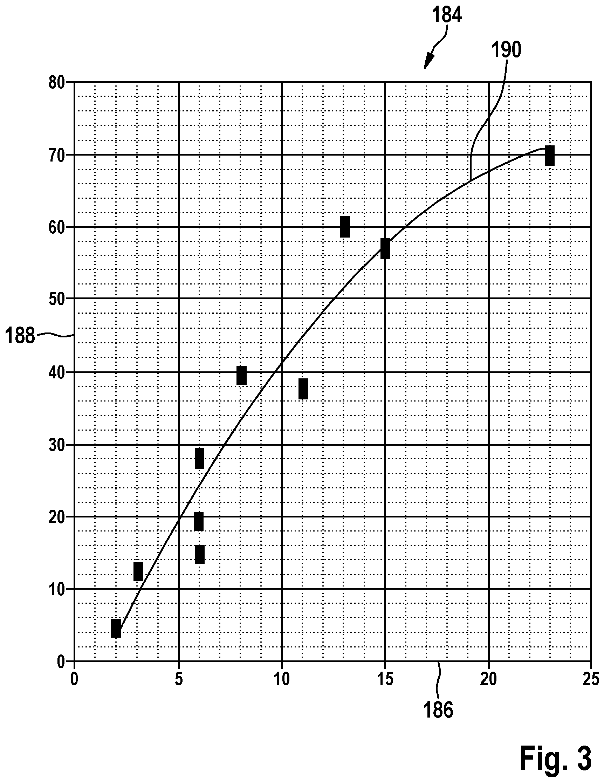

Figur 3 zeigt einen Graphen 184 mit einer Abszisse 186, entlang der eine dynamische Viskosität in mm²/s aufgetragen ist, sowie eine Ordinate 188, entlang der eine Schließdauerdifferenz in µs aufgetragen ist. Innerhalb dieses Diagramms 184 sind mehrere Quadrate für Schließdauerdifferenzen, die sich in Abhängigkeit kinematischer Viskositäten aus Messwerten mit unterschiedlichen Kraftstoffen bei verschiedenen Temperaturen ergeben, dargestellt. Eine Kurve 190 stellt einen Zusammenhang der Messwerte dar.

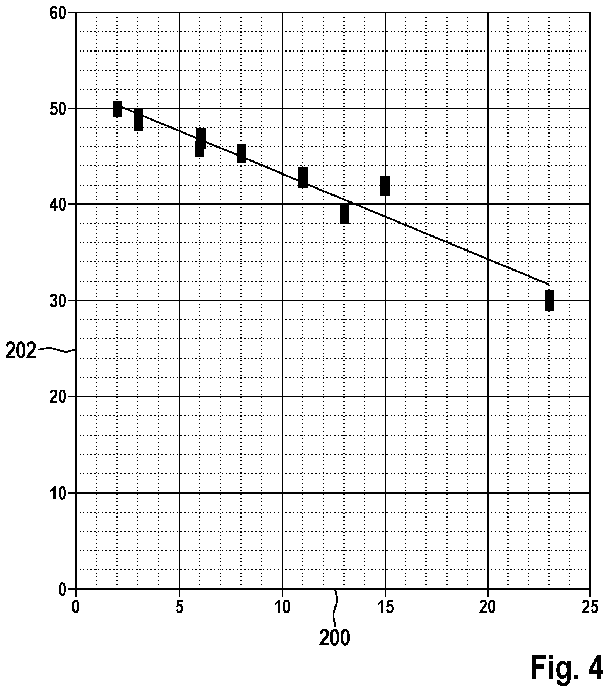

Figur 4 zeigt den Zusammenhang zwischen Einspritzmenge und Viskosität. Dabei ist an einer Abszisse 200 die Viskosität in mm²/s und an einer Ordinate 202 die Einspritzmenge in mm³/Hub aufgetragen.

In Figur 5 ist ein typischer Verlauf eines Sensorstroms dargestellt, um zu verdeutlichen, auf welche Weise z.B. die Schließdauer des Ankers gemessen werden kann.

In der Darstellung ist an einer Ordinate 250 der Sensorstrom gegenüber der Zeit (Abszisse 252) aufgetragen. Die Genauigkeit des eingestellten Sensorstroms hängt im Falle der Boosterung von der Genauigkeit der Boosterspannung ab und von der Genauigkeit, mit der das Steuergerät bei Erreichen einer Abschaltschwelle den Schaltzustand "Boosterung" wieder beendet. Zudem erfolgt mit dem Aufbau des Sensorstroms ein erneuter Anstieg der Magnetkraft des Ventils, der zu einer geringfügigen Verzögerung des Schließvorgangs des Magnetventils führt.

Die Darstellung zeigt in einem ersten Bereich 254 ein Schnelllöschen, bei dem der Messbereich der Spannung überschritten ist. Ein nochmaliges Boostern in einem zweiten Bereich 256 dient zum Aufbau des Sensorstroms, wobei der Messbereich der Spannung überschritten ist. Es erfolgt eine Energieentnahme aus einem Boosterkonsensator, ein sehr schneller Stromanstieg und ein erneuter Magnetkraftanstieg. In einem dritten Bereich 258 ist ein Knick im Stromverlauf zu erkennen, der den Schließzeitpunkt anzeigt.

Figur 6 zeigt in einer schematischen Darstellung eine Anordnung 300 zum Bestimmen mindestens einer Eigenschaft, insbesondere der Viskosität, eines Kraftstoffs. Diese Anordnung umfasst ein Steuergerät 302 mit einer Auswerteelektronik der zur Durchführung der für die vorstehend beschriebenen Verfahren erforderlichen Berechnungsverfahren.

Die Anordnung 300 ist über elektrische Anschlüsse 304 mit einer Einspritzanlage 305, die ein Magnetventil 306 umfasst, als Ventileinheit einer Verbrennungsmaschine 308 gekoppelt. Dieses Magnetventil 306 umfasst einen Anker 310, der zum Schließen und Öffnen des Magnetventils 306 bewegt wird.

Weiterhin zeigt die Darstellung eine Kraftstoffzuleitung 312, durch die Kraftstoff 314 dem Magnetventil 306 zugeführt wird. Der Anker 310 bewegt sich beim Öffnen und Schließen durch diesen Kraftstoff 314, Eigenschaften des Kraftstoffs, insbesondere dessen Viskosität, beeinflussen dabei das dynamische Verhalten des Ankers 310. Es wird nunmehr die Schließdauer gemessen und auf Grundlage der gemessenen Schließdauer ein Faktor ermittelt, der die mindestens eine Eigenschaft, wie bspw. die Viskosität, des Kraftstoffs repräsentiert.

## Patentansprüche

1. Verfahren zum Bestimmen mindestens einer Eigenschaften eines Kraftstoffs (314), bei dem eine Schließdauer eines Ankers (310) eines Magnetventils (306), der sich durch den Kraftstoff (314) bewegt, bei zumindest einer Ansteuerdauer gemessen wird, und aufgrund der gemessenen Schließdauer ein Faktor ermittelt wird, der die mindestens eine Eigenschaft des Kraftstoffs (314) repräsentiert, wobei auf Grundlage der bestimmten mindestens einen Eigenschaft Korrekturwerte für die Ansteuerdauer ermittelt werden.

2. Verfahren nach Anspruch 1, bei dem als Eigenschaft die Viskosität des Kraftstoffs (314) bestimmt wird.

3. Verfahren nach Anspruch 1 oder 2, bei dem mehrere Messungen bei unterschiedlichen Ansteuerdauern durchgeführt werden und aus den gemessenen Schließdauerwerten mindestens ein Vergleichswert ermittelt wird.

4. Verfahren nach einem der Ansprüche 1 bis 3, das vor einem Start des Verbrennungsmotors (308) durchgeführt wird.

5. Verfahren zum Betreiben eines Verbrennungsmotors (308) mit einem Kraftstoff (314), wobei mindestens ein Betriebsparameter einer Einspritzanlage (305) des Verbrennungsmotors (308) in Abhängigkeit mindestens einer Eigenschaft des Kraftstoffs (314), die durch ein Verfahren nach einem der voranstehenden Ansprüche ermittelt wird, eingestellt wird.

6. Verfahren nach Anspruch 5, das zur Durchführung eines Kaltstarts des Verbrennungsmotors (308) angewendet wird.

7. Anordnung zum Bestimmen mindestens einer Eigenschaft eines Kraftstoffs (314), die ein Steuergerät (302) umfasst, das eine Schließdauer eines Ankers (310) eines Magnetventils (306), der sich durch den Kraftstoff (314) bewegt, bei zumindest einer Ansteuerdauer misst und auf Grundlage der gemessenen Schließdauer einen Faktor ermittelt, der die mindestens eine Eigenschaft des Kraftstoffs (314) repräsentiert, wobei auf Grundlage der bestimmten mindestens einen Eigenschaft Korrekturwerte für die Ansteuerdauer ermittelt werden.

8. Anordnung nach Anspruch 7, bei der das Steuergerät (302) dazu ausgebildet ist, einen Betrieb einer Einspritzanlage (305) des Verbrennungsmotors (308), die mindestens ein Magnetventil (306) umfasst, zu kontrollieren und mindestens einen Betriebsparameter der Einspritzanlage (305) in Abhängigkeit der ermittelten mindestens einen Eigenschaft einzustellen.

9. Anordnung nach Anspruch 7 oder 8, die dazu ausgebildet ist, als mindestens eine Eigenschaft die Viskosität des Kraftstoffs (314) zu ermitteln.

## Claims

1. Method for determining at least one property of a fuel (314), in which a closing period of an armature (310) of a solenoid valve (306), which armature (310) moves through the fuel (314), is measured during at least one actuation period, and a factor which represents the at least one property of the fuel (314) is obtained on the basis of the measured closing period, wherein correction values for the actuation period are obtained on the basis of the at least one property which is determined.

2. Method according to Claim 1, in which the viscosity of the fuel (314) is determined as the property.

3. Method according to Claim 1 or 2, in which a plurality of measurements are carried out during different actuation periods, and at least one comparison value is obtained from the measured closing period values.

4. Method according to one of Claims 1 to 3, which is carried out before a start of the internal combustion engine (308).

5. Method for operating an internal combustion engine (308) with a fuel (314), wherein at least one operating parameter of an injection system (305) of the internal combustion engine (308) is set as a function of at least one property of the fuel (314), which property is obtained by means of a method according to one of the preceding claims.

6. Method according to Claim 5 which is applied to carry out a cold start of the internal combustion engine (308).

7. Arrangement for determining at least one property of a fuel (314) which comprises a control unit (302) which measures a closing period of an armature (310) of a solenoid valve (306) which moves through the fuel (314), during at least one actuation period, and obtains, on the basis of the measured closing period, a factor which represents the at least one property of the fuel (314), wherein correction values for the actuation period are obtained on the basis of the at least one property which is determined.

8. Arrangement according to Claim 7, in which the control unit (302) is designed to control operation of an injection system (305) of the internal combustion engine (308) which comprises at least one solenoid valve (306), and to set at least one operating parameter of the injection system (305) as a function of the at least one property which is obtained.

9. Arrangement according to Claim 7 or 8, which is designed to obtain the viscosity of the fuel (314) as at least one property.

## Revendications

1. Procédé pour déterminer au moins une propriété, d'un carburant (314), dans lequel une durée de fermeture d'un induit (310) d'une électrovanne (306) se déplaçant à travers le carburant (314) est mesurée pour au moins une durée de commande et, sur la base de la durée de fermeture mesurée, un facteur représentant ladite au moins une propriété du carburant (314) est déterminé, dans lequel des valeurs de correction pour la durée de commande sont déterminées sur la base de ladite au moins une propriété déterminée.

2. Procédé selon la revendication 1, dans lequel la viscosité du carburant (314) est déterminée en tant que propriété.

3. Procédé selon la revendication 1 ou 2, dans lequel plusieurs mesures sont effectuées pour des durées de commande différentes et au moins une valeur de comparaison est déterminée à partir des valeurs des durées de fermeture mesurées.

4. Procédé selon l'une quelconque des revendications 1 à 3, qui est mis en oeuvre avant un démarrage du moteur à combustion interne (308).

5. Procédé de mise en fonctionnement d'un moteur à combustion interne (308) avec un carburant (314), dans lequel au moins un paramètre de fonctionnement d'une installation d'injection (305) du moteur à combustion interne (308) est réglé en fonction d'au moins une propriété du carburant (314) qui est déterminée par un procédé selon l'une quelconque des revendications précédentes.

6. Procédé selon la revendication 5, qui est utilisé pour mettre en oeuvre un démarrage à froid du moteur à combustion interne (308).

7. Dispositif destiné à déterminer au moins une propriété d'un carburant (314), comprenant un appareil (302) qui mesure une durée de fermeture d'un induit (310) d'une électrovanne (306) se déplaçant à travers le carburant (314) pour au moins une durée de commande et détermine, sur la base de la durée de fermeture mesurée, un facteur représentant ladite au moins une propriété du carburant (314), dans lequel des valeurs de correction pour la durée de commande sont déterminées sur la base de ladite au moins une propriété déterminée.

8. Dispositif selon la revendication 7, dans lequel l'appareil de commande (302) est conçu pour contrôler un fonctionnement d'une installation d'injection (305) du moteur à combustion interne (308), qui comprend au moins une électrovanne (306), et pour régler au moins un paramètre de fonctionnement de l'installation d'injection (305) en fonction de ladite au moins une propriété déterminée.

9. Dispositif selon la revendication 7 ou 8, conçu pour déterminer la viscosité du carburant (314) en tant que ladite au moins une propriété.
